# EUROPÄISCHE PATENTSCHRIFT

(11) **EP 1 464 385 B1**
(45) Veröffentlichungstag und Bekanntmachung des Hinweises auf die Patenterteilung: **21.01.2009**
(21) Anmeldenummer: 03007730.9
(22) Anmeldetag: 04.04.2003
(51) Int. Cl.: B01J 13/02, D06M 23/12, A61K 8/11

(54) **Mikrokapseln (XVIII)**
Microcapsules (XVIII)
Microcapsules (XVIII)

(43) Veröffentlichungstag der Anmeldung: 06.10.2004
(73) Patentinhaber: Cognis IP Management GmbH, 40589 Düsseldorf (DE)
(72) Erfinder: Viladot Petit, Joseph-Llius, Dr., 08018 Barcelona (ES); Domingo, Marta, 08024 Barcelona (ES); de Moragas, Maria, Dr., 08310 Argentona (Barcelona) (ES)

(56) Entgegenhaltungen:
- EP-A- 1 243 320
- EP-A- 1 243 325

## Beschreibung

### Gebiet der Erfindung

Die Erfindung befindet sich auf dem Gebiet der verkapselten Wirkstoffe und betrifft neue Mikrokapseln mit einer inneren Wachsphase, ein Verfahren zu deren Herstellung sowie deren Verwendung im Bereich der Kosmetik und Textiltechnik.

### Stand der Technik

Unter dem Begriff "Mikrokapsel" werden vom Fachmann sphärische Aggregate mit einem Durchmesser im Bereich von etwa 0,0001 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide). Chitosanmikrokapseln und Verfahren zu ihrer Herstellung sind Gegenstand früherer Patenanmeldungen der Patentanmelderin **[**WO 01/01926**,** WO 01/01927**,** WO 01/01928**,** WO 01101929**].**

Insbesondere aus der WO 01/66240 (Cognis) sind Hautpflegemittel bekannt, die mit Retinol oder Retinolsäure beladene Mikrokapseln mit mittleren Durchmessern im Bereich von 0,1 bis 5 mm, enthalten. Die Kapseln werden erhalten, indem man
(a) wässrige Zubereitungen von Retinol oder Retinolsäure mit Ölkörpern in Gegenwart von Emulgatoren zu O/W-Emulsionen verarbeitet,
(b) die so erhaltenen Emulsionen mit wässrigen Lösungen anionischer Polymere behandelt,
(c) die so erhaltene Matrix zur Ausbildung einer Hülle mit wässrigen Chitosanlösungen in Kontakt bringt und
(d) die so erhaltenen Verkapselungsprodukte, gegebenenfalls in Gegenwart von Verdickungsmitteln, von der wässrigen Phase abtrennt.

Obschon die auf diese Weise verkapselten Wirkstoffen bereits gegenüber Produkten des Stands der Technik eine wesentlich höhere Stabilität aufweisen, besteht nach wie vor ein besonderes Interesse, sowohl die Photostabilität empfindlicher Wirkstoffe, speziell des Retinols, als auch die mechanische Härte der Kapseln zu verbessern. Die Aufgabe der vorliegenden Erfindung hat somit darin bestanden, neue Mikrokapseln zur Verfügung zu stellen, welche diese Anforderungen erfüllen.

### Beschreibung der Erfindung

Gegenstand der Erfindung sind neue Mikrokapseln mit mittleren Durchmessern im Bereich von 0,0001 bis 5 mm, bestehend aus einer Hüllmembran und einer die Wirkstoffe enthaltenden Matrix, welche dadurch erhältlich sind, dass man
(a1) fettlösliche Wirkstoffe mit Schmelzpunkten unterhalb von 20 °C und Wachse mit Schmelzpunkten oberhalb von 20 °C zu einer Ölphase verarbeitet,
(a2) die so erhaltene Ölphase mit einer wässrigen Lösung eines Anionpolymers bei einer Temperatur oberhalb des Schmelzpunktes der Wachse vermischt, und
(a3) die so erhaltene O/W-Emulsion auf eine Temperatur unterhalb des Schmelzpunktes der Wachse abkühlt und mit der wässrigen Lösung eines Kationpolymers in Kontakt bringt,
oder
(b1) fettlösliche Wirkstoffe mit Schmelzpunkten unterhalb von 20 °C und Wachse mit Schmelzpunkten oberhalb von 20 °C zu einer Ölphase verarbeitet,
(b2) die so erhaltene Ölphase mit einer wässrigen Lösung eines Kationpolymers bei einer Temperatur oberhalb des Schmelzpunktes der Wachse vermischt, und
(b3) die so erhaltene O/W-Emulsion auf eine Temperatur unterhalb des Schmelzpunktes der Wachse abkühlt und mit der wässrigen Lösung eines Anionpolymers in Kontakt bringt.

Überraschenderweise wurde gefunden, dass das Einbetten der Wirkstoffe in eine Wachsmatrix nicht nur zu einer gesteigerten mechanischen Stabilität der Mikrokapseln führt, sondern auch die Photostabilität der empfindlichen Inhaltsstoffe signifikant verbessert.

Ein weiterer Gegenstand der Erfindung betrifft ein Verfahren zur Herstellung von Mikrokapseln mit mittleren Durchmessern im Bereich von 0,0001 bis 5 mm, bestehend aus einer Hüllmembran und einer die Wirkstoffe enthaltenden Matrix, bei dem man
(a1) fettlösliche Wirkstoffe mit Schmelzpunkten unterhalb von 20 °C und Wachse mit Schmelzpunkten oberhalb von 20 °C zu einer Ölphase verarbeitet,
(a2) die so erhaltene Ölphase mit einer wässrigen Lösung eines Anionpolymers bei einer Temperatur oberhalb des Schmelzpunktes der Wachse vermischt, und
(a3) die so erhaltene O/W-Emulsion mit der wässrigen Lösung eines Kationpolymers in Kontakt bringt,
oder
(b1) fettlösliche Wirkstoffe mit Schmelzpunkten unterhalb von 20 °C und Wachse mit Schmelzpunkten oberhalb von 20 °C zu einer Ölphase verarbeitet,
(b2) die so erhaltene Ölphase mit einer wässrigen Lösung eines Kationpolymers bei einer Temperatur oberhalb des Schmelzpunktes der Wachse vermischt, und
(b3) die so erhaltene O/W-Emulsion mit der wässrigen Lösung eines Anionpolymers in Kontakt bringt.

### Fettlösliche Wirkstoffe

Als fettlösliche Wirkstoffe, die bei 20 °C in flüssiger Form vorliegen, kommen beispielsweise Retinolsäure, Bisabolol, Panthenol, die diversen Tocopherole und Carotinoide sowie Flavonoide in Frage. Bevorzugt ist der Einsatz von Retinol, da dieser Wirkstoff besondere Anwendung im Bereich der Kosmetik findet, jedoch besonders leicht photochemisch abgebaut wird.

### Wachse

Als Wachse, die als stabilisierende Phase für die Wirkstoffe dienen, kommen in erster Linie gehärtete Fette, Fettalkohole, Wachsester sowie Sterole in Frage, soweit diese die Anforderung erfüllen, dass sie bei 20 °C in fester Form vorliegen und erst oberhalb dieser Temperatur, üblicherweise oberhalb von 30 °C schmelzen. Typische Beispiele sind die gesättigten Fettalkohole mit 16 bis 22 Kohlenstoffatomen, also Cetylalkohol, Stearylalkohol, Cetearylalkohol und Behenylalkohol. Als Wachsester eignen sich die Kondensationsprodukte dieser Alkohole mit den entsprechenden korrespondierenden Fettsäuren, also beispielsweise Palmitylpalmitat, Cetearylstearat, oder Behenylpalmitat. Unter den Sterolen kommen insbesondere die natürlichen Phytosterole in Frage. Grundsätzlich können auch natürliche Wachse eingesetzt werden, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin oder Ozokerit (Erdwachs). Da diese jedoch einen vergleichsweise hohen Schmelzpunkt haben wären zur Herstellung der Ölphase hohe Temperaturen erforderlich, was nicht nur technisch aufwendiger und kostspieliger wäre, sondern auch zu einer Schädigung der Wirkstoffe führen könnte. Gleiches gilt für petrochemische Wachse, wie z.B. Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse mit Molgewichten im Bereich von 200 bis 1000. Nichtsdestotrotz kann es im Einzelfall Sinn machen, auf diese hochschmelzenden Wachse auszuweichen.

### Anionpolymere

Die anionische Polymere haben die Aufgabe, mit den kationischen Polymeren Membranen zu bilden. Für diesen Zweck eignen sich vorzugsweise Salze der Alginsäure. Bei der Alginsäure handelt es sich um ein Gemisch carboxylgruppenhaltiger Polysaccharide mit folgendem idealisierten Monomerbaustein:

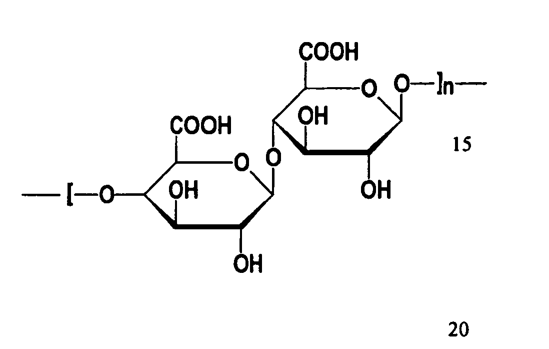

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.

### Gelbildner

Zur Formung der Wachsmatrix kann es mitunter nützlich sein, Gelbildner mitzuverwenden, welche vorzugsweise zusammen mit den Polymeren über die wässrige Phase eingebracht werden. Im Sinne der Erfindung werden als Gelbildner vorzugsweise solche Stoffe in Betracht gezogen, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3-und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

### Emulgatoren

Ferner hat es sich als vorteilhaft erwiesen, die Bildung der O/W-Emulsion durch die Mitverwendung von geeigneten Emulgatoren zu unterstützen, welche ebenfalls vorzugsweise über die wässrige Phase eingebracht werden. Für diesen Zweck kommen insbesondere nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

### ➢ Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

### ➢ Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### ➢ Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

### ➢ Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### ➢ Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### ➢ Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### ➢ Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine - COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Kationische Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol und ungesättigten Tetraalkylammoniumverbindungen, wie z.B. Polyquatampho® 149 (Cognis)

In einer bevorzugten Ausführungsform der Erfindung werden zur Ausbildung der Hüllmembran kationische Polymere vom Typ der Chitosane eingesetzt. Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgendenidealisierten - Monomerbaustein enthalten:

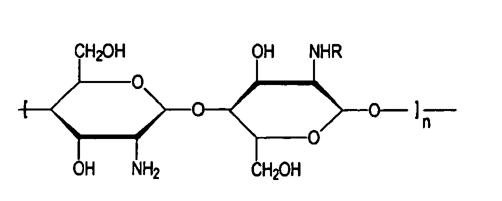

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

### Herstellverfahren Mikrokapseln

Die Herstellung der Mikrokapseln erfolgt in mehreren Schritten. Zunächst wird eine Ölphase hergestellt, indem man das Wachs über seinen Schmelzpunkt auf beispielsweise 60 bis 80 °C erwärmt und den bei Raumtemperatur flüssigen fettlöslichen Wirkstoff darin löst. Üblicherweise werden dabei Wirkstoffe und Wachsen im Gewichtsverhältnis 3 : 7 bis 0,5 : 9,5 und vorzugsweiswe 2 : 8 bis 1 : 9 eingesetzt. Parallel wird eine wässrige Phase vorbereitet, welche neben dem anionischen Polymer auch noch - falls gewünscht - einen Gelbildner und/oder Emulgator enthalten kann. Üblicherweise enthält die wässrige Phase 1 bis 5 Gew.-% Polymer sowie jeweils 1 bis 2 Gew.-% der beiden anderen Komponenten. Die beiden Phasen werden als dann oberhalb des Schmelzpunktes der Wachse unter intensivem Rühren miteinander vermischt, wobei eine W/O-Emulsion entsteht. Zur Ausbildung der Hüllmembran wird die Emulsion zunächst auf eine Temperatur unterhalb des Schmlezpunktes der Wachse abgekühlt und dann portionsweise mit einer 1 bis 5 Gew.-%igen wässrigen Lösung des Kationpolymers versetzt. Anion- und Kationpolymer sind dabei in den Verfahrensschritten austauschbar, d.h. die Emulsionsbildung kann auch unter Einsatz des kationischen Polymers erfolgen, während die Hülle dann durch Fällung mit dem Anionpolymer erzeugt wird. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert gewöhnlich auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und -diesten von Fettsäuren, Polyacrylaten, Polyacrylamiden und dergleichen noch unterstützt werden kann. Abschließend können die Mikrokapseln - falls gewünscht - von der wässrigen Phase beispielsweise durch Dekantieren, Filtrieren oder Zentrifugieren abgetrennt werden. Auf diesem Wege werden Mikrokapseln erhalten, welche im Mittel einen Durchmesser im Bereich von vorzugsweise etwa 0,1 bis 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig.

### Gewerbliche Anwendbarkeit

Die erfindungsgemäßen neuen Mikrokapseln eignen sich in besonderer Weise zur Stabilisierung empfindlicher Wirkstoffe, wie sie beispielsweise im Bereich der Kosmetik, speziell der Hautpflege sowie zur pflegenden Ausrüstung von Textilien eingesetzt werden. Weitere Gegenstände der vorliegenden Erfindung betreffen daher ihre Verwendung zur Herstellung von kosmetischen Zubereitungen sowie zur Ausrüstung von Textilien, wobei deren Einsatzmengen 0,1 bis 50, vorzugsweise 1 bis 30 und insbesondere 2 bis 10 Gew.-% betragen können.

### Kosmetische Zubereitungen

Die erfindungsgemäßen neuen Mikrokapseln können zur Herstellung von kosmetischen Zubereitungen, wie beispielsweise Haarshampoos, Haarlotionen, Schaumbäder, Duschbäder, Cremes, Gele, Lotionen, alkoholischen und wässrig/alkoholischen Lösungen, Emulsionen, Wachs/ Fett-Massen, Stiftpräparaten, Pudern und dergleichen dienen. Diese Mittel können als weitere Hilfs- und Zusatzstoffe neben den bereits eingangs aufgelisteten Emulgatoren ferner milde Tenside, Ölkörper, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. DE 19756377 A1), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind die bereits eingangs aufgelisteten Stoffe. Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfilter

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher beschrieben;
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-isopropylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze; ;
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

### Biogene Wirkstoffe und Antioxidantien

Unter biogenen Wirkstoffen - welche, soweit sie fettlöslich sind, mit den zu verkapselnden Wirkstoffen identisch sein können, sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

### ➢ Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

### ➢ Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### ➢ Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

### ➢ Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. A-luminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel, Insektenrepellentien, Selbstbräuner und Depigmentierungsmittel

Als Quellmittel für wässrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993**)** entnommen werden. Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedem-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Stemanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.1.42051), Indigotin (C.1.73015), Chlorophyllin (C.1.75810), Chinolingelb (C.1.47005), Titandioxid (C.1.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-%-bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

### Beispiel 1

In einer Rührapparatur wurden 10 g einer 10 Gew.-%igen Lösung von Retinol in Sojaöl (Retinol® S20, BASF), 10 g Cetearylalkohol (Lanette O, Cognis) und 0,5 g Sorbitanmonostearat+20EO (Eumulgin® SMS 20, Cognis) vorgelegt, bei 80 °C aufgeschmolzen und homogenisiert. Anschließend wurde die Zubereitung unter intensivem Rühren mit 50 g einer 2 Gew.-%igen wässrigen Calciumalginatlösung vermischt. Die entstandene O/W-Emulsion wurde auf 50 °C abgekühlt und schließlich portionsweise mit solchen Menge einer 1 Gew.-%igen Lösung von Chitosan in Glycolsäure (Hydagen® CMF Cognis) versetzt, dass sich eine Chitosankonzentration von 0,075 Gew.-% - bezogen auf die Zubereitung - einstellte. Schließlich wurde der pH-Wert durch Zugabe von Triethanolamin auf 5,5 angehoben.

### Beispiel 2

In einer Rührapparatur wurden 10 g einer 10 Gew.-%igen Lösung von Retinol in Sojaöl (Retinol® S20, BASF), 10 g Phytosterol (Generol 122 N, Cognis) und 0,5 g Sorbitanmonostearat+20EO (Eumulgin® SMS 20, Cognis) vorgelegt, bei 80 °C aufgeschmolzen und homogenisiert. Anschließend wurde die Zubereitung unter intensivem Rühren mit 50 g einer 2 Gew.-%igen wässrigen Calciumalginatlösung vermischt. Die entstandene O/W-Emulsion wurde auf 50 °C abgekühlt und schließlich portionsweise mit solchen Menge einer 1 Gew.-%igen Lösung von Chitosan in Glycolsäure (Hydagen® CMF Cognis) versetzt, dass sich eine Chitosankonzentration von 0,075 Gew.-% - bezogen auf die Zubereitung - einstellte. Schließlich wurde der pH-Wert durch Zugabe von Triethanolamin auf 5,5 angehoben.

### Beispiel 3

In einer Rührapparatur wurden 10 g einer 10 Gew.-%igen Lösung von Retinol in Sojaöl (Retinol® S20, BASF), 10 g Cetearylalkohol und 0,5 g Sorbitanmonostearat+20EO (Eumulgin® SMS 20, Cognis) vorgelegt, bei 80 °C aufgeschmolzen und homogenisiert. Anschließend wurde die Zubereitung unter intensivem Rühren mit 50 g einer 2 Gew.-%igen wässrigen Polyacrylatlösung (Molgewicht ca. 10.000) vermischt. Die entstandene O/W-Emulsion wurde auf 50 °C abgekühlt und schließlich portionsweise mit solchen Menge einer 1 Gew.-%igen Lösung von Chitosan in Glycolsäure (Hydagen® CMF Cognis) versetzt, dass sich eine Chitosankonzentration von 0,075 Gew.-% - bezogen auf die Zubereitung - einstellte. Schließlich wurde der pH-Wert durch Zugabe von Triethanolamin auf 5,5 angehoben.

### Beispiel 4

In einer Rührapparatur wurden 10 g einer 10 Gew.-%igen Lösung von Retinol in Sojaöl (Retinol® S20, BASF), 10 g Cetearylalkohol (Lanette O, Cognis) und 0,5 g Sorbitanmonostearat+20EO (Eumulgin® SMS 20, Cognis) vorgelegt, bei 80 °C aufgeschmolzen und homogenisiert. Anschließend wurde die Zubereitung unter intensivem Rühren mit 50 g einer 1 Gew.-%igen wässrigen Chitosanlösung vermischt. Die entstandene O/W-Emulsion wurde auf 50 °C abgekühlt und schließlich portionsweise mit solchen Menge einer 2 Gew.-%igen Calciumalginatlösung versetzt, dass sich eine Alginatkonzentration von 0,075 Gew.-% - bezogen auf die Zubereitung - einstellte. Schließlich wurde der pH-Wert durch Zugabe von Triethanolamin auf 5,5 angehoben.

In der folgenden Tabelle 2 finden sich eine Reihe von Formulierungsbeispielen.

**Tabelle 2**

| **Beispiele für kosmetische Zubereitungen (Wasser, Konservierungsmittel ad 100 Gew.-%)** | | | | | | | | | | |
|---|---|---|---|---|---|---|---|---|---|---|
| **Zusammensetzung (INCI)** | **1** | **2** | **3** | **4** | **5** | **6** | **7** | **8** | **9** | **10** |
| **Texapon**® **NSO** | - | - | - | - | - | - | 38,0 | 38,0 | 25,0 | - |
| Sodium Laureth Sulfate | | | | | | | | | | |
| **Texapon**® **SB 3** | - | - | - | - | - | - | - | - | 10,0 | - |
| Disodium Laureth Sulfosuccinate | | | | | | | | | | |
| **Plantacare**® **818** | - | - | - | - | - | - | 7,0 | 7,0 | 6,0 | - |
| Coco Glucosides | | | | | | | | | | |
| **Plantacare**® **PS 10** | - | - | - | - | - | - | - | - | - | 16,0 |
| Sodium Laureth Sulfate (and) Coco Glucosides | | | | | | | | | | |
| **Dehyton**® **PK 45** | - | - | - | - | - | - | - | - | 10,0 | - |
| Cocamidopropyl Betaine | | | | | | | | | | |
| **Dehyquart**® **A** | 2,0 | 2,0 | 2,0 | 2,0 | 4,0 | 4,0 | - | - | - | - |
| Cetrimonium Chloride | | | | | | | | | | |
| **Dehyquart L**® **80** | 1,2 | 1,2 | 1,2 | 1,2 | 0,6 | 0,6 | - | - | - | - |
| Dicocoylmethylethoxymonium Methosulfate (and) Propylenglycol | | | | | | | | | | |
| **Eumulgin**® **B2** | 0,8 | 0,8 | - | 0,8 | - | 1,0 | - | - | - | - |
| Ceteareth-20 | | | | | | | | | | |
| **Eumulgin**® **VL 75** | - | - | 0,8 | - | 0.8 | - | - | - | - | - |
| Lauryl Glucoside (and) Polyglyceryl-2 Polyhydroxystearate (and) Glycerin | | | | | | | | | | |
| **Lanette**® **O** | 2,5 | 2,5 | 2,5 | 2,5 | 3,0 | 2,5 | - | - | - | - |
| Cetearyl Alcohol | | | | | | | | | | |
| **Cutina**® **GMS** | 0,5 | 0,5 | 0,5 | 0,5 | 0,5 | 1,0 | - | - | - | - |
| Glyceryl Stearate | | | | | | | | | | |
| **Cetiol**® **HE** | 1,0 | - | - | - | - | - | - | - | 1,0 | |
| PEG-7 Glyceryl Cocoate | | | | | | | | | | |
| **Cetiol**® **PGL** | - | 1,0 | - | - | 1,0 | - | - | - | - | - |
| Hexyldecanol (and) Hexyldecyl Laurate | | | | | | | | | | |
| **Cetiol**® **V** | - | - | - | 1,0 | - | - | - | - | - | - |
| Decyl Oleate | | | | | | | | | | |
| **Eutanol**® **G** | - | - | 1,0 | - | - | 1,0 | - | - | - | - |
| Octyldodecanol | | | | | | | | | | |
| **Nutrilan**® **Keratin W** | - | - | - | 2,0 | - | - | - | - | - | - |
| Hydrolyzed Keratin | | | | | | | | | | |
| **Lamesoft**® **LMG** | - | - | - | - | - | - | 3,0 | 2,0 | 4,0 | - |
| Glyceryl Laurate (and) Potassium Cocoyl Hydrolyzed Collagen | | | | | | | | | | |
| **Euperlan**® **PK 3000 AM** | - | - | - | - | - | - | - | 3,0 | 5,0 | 5,0 |
| Glycol Distearate (and) Laureth-4 (and) Cocamidopropyl Betaine | | | | | | | | | | |
| **Generol**® **122 N** | - | - | - | - | - | - | - | - | - | - |
| Soja Sterol | | | | | | | | | | |
| Retinoi-Mikrokapseln nach Beispiel 1 | 1,0 | 1,0 | 1,0 | 1,0 | 1,0 | 1,0 | 1,0 | 1,0 | 1,0 | 1,0 |
| **Hydagen**® **CMF** | 1,0 | 1,0 | 1,0 | 1,0 | 1,0 | 1,0 | 1,0 | 1,0 | 1,0 | 1,0 |
| Chitosan | | | | | | | | | | |
| **Copherol**® **1250** | - | - | 0,1 | 0,1 | - | - | - | - | - | - |
| Tocopherol Acetate | | | | | | | | | | |
| **Arlypon**® **F** | - | - | - | - | - | - | 3,0 | 3,0 | 1,0 | - |
| Laureth-2 | | | | | | | | | | |
| **Sodium Chloride** | - | - | - | - | - | - | - | 1,5 | - | 1,5 |

| | | | | | | | | | | |
|---|---|---|---|---|---|---|---|---|---|---|
| (1-4) Haarspülung, (5-6) Haarkur, (7-8) Duschbad, (9) Duschgel, (10) Waschlotion | | | | | | | | | | |

**Tabelle 2**

| **Beispiele für kosmetische Zubereitungen (Wasser, Konservierungsmittel ad 100 Gew.-%) - Forts.** | | | | | | | | | | |
|---|---|---|---|---|---|---|---|---|---|---|
| **Zusammensetzung (INCI)** | **11** | **12** | **13** | **14** | **15** | **16** | **17** | **18** | **19** | **20** |
| **Texapon**® **NSO** | 20,0 | 20,0 | 12,4 | - | 25,0 | 11,0 | - | - | - | - |
| Sodium Laureth Sulfate | | | | | | | | | | |
| **Texapon**® **K 14 S** | - | - | - | - | - | - | - | - | 11,0 | 23,0 |
| Sodium Myreth Sulfate | | | | | | | | | | |
| **Texapon**® **SB 3** | - | - | - | - | - | 7,0 | - | - | - | - |
| Disodium Laureth Sulfosuccinate | | | | | | | | | | |
| **Plantacare**® **818** | 5,0 | 5,0 | 4,0 | - | - | - | - | - | 6,0 | 4,0 |
| Coco Glucosides | | | | | | | | | | |
| **Plantacare**® **2000** | - | - | - | - | 5,0 | 4,0 | - | - | - | - |
| Decyl Glucoside | | | | | | | | | | |
| **Plantacare**® **PS 10** | - | - | - | 40,0 | - | - | 16,0 | 17,0 | - | - |
| Sodium Laureth Sulfate (and) Coco Glucosides | | | | | | | | | | |
| **Dehyton**® **PK 45** | 20,0 | 20,0 | - | - | 8,0 | - | - | - | - | 7,0 |
| Cocamidopropyl Betaine | | | | | | | | | | |
| **Eumulgin**® **BI** | - | - | - | - | 1,0 | - | - | - | - | - |
| Ceteareth-12 | | | | | | | | | | |
| **Eumulgin**® **B2** | - | - | - | 1,0 | - | - | - | - | - | - |
| Ceteareth-20 | | | | | | | | | | |
| **Lameform**® **TGI** | - | - | - | 4,0 | - | - | - | - | - | - |
| Polyglyceryl-3 Isostearate | | | | | | | | | | |
| **Dehymuls**® **PGPH** | - | - | 1,0 | - | - | - | - | - | - | - |
| Polyglyceryl-2 Dipolyhydroxystearate | | | | | | | | | | |
| **Monomuls**® **90-L 12** | - | - | - | - | - | - | - | - | 1,0 | 1,0 |
| Glyceryl Laurate | | | | | | | | | | |
| **Cetiol**® **HE** | - | 0,2 | - | - | - | - | - | - | - | - |
| PEG-7 Glyceryl Cocoate | | | | | | | | | | |
| **Eutanol**® **G** | - | - | - | 3,0 | - | - | - | - | - | - |
| Octyldodecanol | | | | | | | | | | |
| **Nutrilan**® **Keratin W** | - | - | - | - | - | - | - | - | 2,0 | 2,0 |
| Hydrolyzed Keratin | | | | | | | | | | |
| Nutrilan® I | 1,0 | - | - | - | - | 2,0 | - | 2,0 | - | - |
| Hydrolyzed Collagen | | | | | | | | | | |
| **Lamesoft**® **LMG** | - | - | - | - | - | - | - | - | 1,0 | - |
| Glyceryl Laurate (and) Potassium Cocoyl Hydrolyzed Collagen | | | | | | | | | | |
| **Lamesoft**® **156** | - | - | - | - | - | - | - | - | - | 5,0 |
| Hydrogenated Tallow Gyceride (and) Potassium Cocoyl | | | | | | | | | | |
| Hydrolyzed Collagen | | | | | | | | | | |
| **Gluadin**® **WK** | 1,0 | 1,5 | 4,0 | 1,0 | 3,0 | 1,0 | 2,0 | 2,0 | 2,0 | - |
| Sodium Cocoyl Hydrolyzed Wheat Protein | | | | | | | | | | |
| **Euperlan**® **PK 3000 AM** | 5,0 | 3,0 | 4,0 | - | - | - | - | 3,0 | 3,0 | - |
| Glycol Distearate (and) Laureth-4 (and) Cocamidopropyl Betaine | | | | | | | | | | |
| **Arlypon**® **F** | 2,6 | 1,6 | - | 1,0 | 1,5 | - | - | - | - | - |
| Laureth-2 | | | | | | | | | | |
| **Retinol-Mikrokapsein** nach Beispiel 1 | 1,0 | 1,0 | 1,0 | 1,0 | 1,0 | 1,0 | 1,0 | 1,0 | 1,0 | 1,0 |
| **Hydagen**® **CMF** | 1,0 | 1,0 | 1,0 | 1,0 | 1,0 | 1,0 | 1,0 | 1,0 | 1,0 | 1,0 |
| Chitosan | | | | | | | | | | |
| **Sodium Chloride** | - | - | - | - | - | 1,6 | 2,0 | 2,2 | - | 3,0 |
| **Glycerin** (86 Gew.-%ig) | - | 5,0 | - | - | - | - | - | 1,0 | 3,0 | - |

| | | | | | | | | | | |
|---|---|---|---|---|---|---|---|---|---|---|
| (11-14) Duschbad "Two-in-One), (15-20) Shampoo | | | | | | | | | | |

**Tabelle 2**

| **Beispiele für kosmetische Zubereitungen (Wasser, Konservierungsmittel ad 100 Gew.-%) - Forts.** | | | | | | | | | | |
|---|---|---|---|---|---|---|---|---|---|---|
| **Zusammensetzung (INCI)** | **21** | **22** | **23** | **24** | **25** | **26** | **27** | **28** | **29** | **30** |
| **Texapon**® NSO | - | 30,0 | 30,0 | - | 25,0 | - | - | - | - | - |
| Sodium Laureth Sulfate | | | | | | | | | | |
| **Plantacare**® **818** | - | 10,0 | - | - | 20,0 | - | - | - | - | - |
| Coco Glucosides | | | | | | | | | | |
| **Plantacare**® **PS 10** | 22,0 | - | 5,0 | 22,0 | - | - | - | - | - | - |
| Sodium Laureth Sulfate (and) Coco Glucosides | | | | | | | | | | |
| **Dehyton**® **PK 45** | 15,0 | 10,0 | 15,0 | 15,0 | 20,0 | - | - | - | - | - |
| Cocamidopropyl Betaine | | | | | | | | | | |
| **Emulgade**® **SE** | - | - | - | - | - | 5,0 | 5,0 | 4,0 | - | - |
| Glyceryl Sterate (and) Ceteareth 12/20 (and) Cetearyl Alcohol | | | | | | | | | | |
| (and) Cetyl Palmitate | | | | | | | | | | |
| **Eumulgin**® **B1** | - | - | - | - | - | - | - | 1,0 | - | - |
| Ceteareth-12 | | | | | | | | | | |
| **Lameform**® **TGI** | - | - | - | - | - | - | - | - | 4,0 | - |
| Polyglyceryl-3 Isostearate | | | | | | | | | | |
| **Dehymuls**® **PGPH** | - | - | - | - | - | - | - | - | - | 4,0 |
| Polyglyceryl-2 Dipolyhydroxystearate | | | | | | | | | | |
| **Monomuls**® **90-O 18** | - | - | - | - | - | - | - | - | 2,0 | - |
| Glyceryl Oleate | | | | | | | | | | |
| **Cetiol**® HE | 2,0 | - | - | 2,0 | 5,0 | - | - | - | - | 2,0 |
| PEG-7 Glyceryl Cocoate | | | | | | | | | | |
| **Cetiol**® **OE** | - | - | - | - | - | - | - | - | 5,0 | 6,0 |
| Dicaprylyl Ether | | | | | | | | | | |
| **Cetiol**® **PGL** | - | - | - | - | - | - | - | 3,0 | 10,0 | 9,0 |
| Hexyldecanol (and) Hexyldecyl Laurate | | | | | | | | | | |
| **Cetiot**® **SN** | - | - | - | - | - | 3,0 | 3,0 | - | - | - |
| Cetearyl Isononanoate | | | | | | | | | | |
| **Cetiol**® **V.** | - | - | - | - | - | 3,0 | 3,0 | - | - | - |
| Decyl Oleate | | | | | | | | | | |
| **Myritol**® **318** | - | - | - | - | - | - | - | 3,0 | 5,0 | 5,0 |
| Coco Caprylate Caprate | | | | | | | | | | |
| **Bees Wax** | - | - | - | - | - | - | - | - | 7,0 | 5,0 |
| **Nutrilan**® **Elastin E20** | - | - | - | - | - | 2,0 | - | - | - | - |
| Hydrolyzed Elastin | | | | | | | | | | |
| **Nutrilan**® I-50 | - | - | - | - | 2,0 | - | 2,0 | - | - | - |
| Hydrolyzed Collagen | | | | | | | | | | |
| **Gluadin**® **AGP** | 0,5 | 0,5 | 0,5 | - | - | - | - | 0,5 | - | - |
| Hydrolyzed Wheat Gluten | | | | | | | | | | |
| **Gluadin**® **WK** | 2,0 | 2,0 | 2,0 | 2,0 | 5,0 | - | - | - | 0,5 | 0,5 |
| Sodium Cocoyl Hydrolyzed Wheat Protein | | | | | | | | | | |
| **Euperlan**® **PK 3000 AM** | 5,0 | - | - | 5,0 | - | - | - | - | - | - |
| Glycol Distearate (and) Laureth-4 (and) Cocamidopropyl Betaine | | | | | | | | | | |
| **Arlypon**® **F** | - | - | - | - | - | - | - | - | - | - |
| Laureth-2 | | | | | | | | | | |
| Retinol-Mikrokapseln nach Beispiel 1 | 1,0 | 1,0 | 1,0 | 1,0 | 1,0 | 1,0 | 1,0 | 1,0 | 1,0 | 1,0 |
| **Hydagen**® **CMF** | 1,0 | 1,0 | 1,0 | 1,0 | 1,0 | 1,0 | 1,0 | 1,0 | 1,0 | 1,0 |
| Chitosan | | | | | | | | | | |
| **Magnesium Sulfate Hepta Hydrate** | - | - | - | - | - | - | - | - | 1,0 | 1,0 |
| **Glycerin** (86 Gew.%ig) | - | - | - | - | - | 3,0 | 3,0 | 5,0 | 5,0 | 3,0 |

| | | | | | | | | | | |
|---|---|---|---|---|---|---|---|---|---|---|
| (21-25) Schaumbad, (26) Softcreme, (27, 28) Feuchtigkeitsemulsion, (29, 30) Nachtcreme | | | | | | | | | | |

**Tabelle 2**

| **Beispiele für kosmetische Zubereitungen (Wasser, Konservierungsmittel ad 100 Gew.-%) - Forts.** | | | | | | | | | | |
|---|---|---|---|---|---|---|---|---|---|---|
| **Zusammensetzung (INCI)** | **31** | **32** | **33** | **34** | **35** | **36** | **37** | **38** | **39** | **40** |
| **Dehymuls**® **PGPH** | 4,0 | 3,0 | - | 5,0 | - | - | - | - | - | - |
| Polyglyceryl-2 Dipolyhydroxystearate | | | | | | | | | | |
| **Lameform**® **TGI** | 2,0 | 1,0 | - | - | - | - | - | - | - | - |
| Polyglyceryl-3 Diisostearate | | | | | | | | | | |
| **Emulgade**® **PL 68/50** | - | - | - | - | 4,0 | - | - | - | 3,0 | - |
| Cetearyl Glucoside (and) Cetearyl Alcohol | | | | | | | | | | |
| **Eumulgin**®**B2** | - | - | - | - | - | - | - | 2,0 | - | - |
| Ceteareth-20 | | | | | | | | | | |
| **Tegocare**® **PS** | - | - | 3,0 | - | - | - | 4,0 | - | - | - |
| Polyglyceryl-3 Methylglucose Distearate | | | | | | | | | | |
| Eumulgin VL 75 | - | - | - | - | - | 3,5 | - | - | 2,5 | - |
| Polyglyceryl-2 Dipolyhydroxystearate (and) Lauryl Glucoside | | | | | | | | | | |
| (and) Glycerin | | | | | | | | | | |
| Bees Wax | 3,0 | 2,0 | 5,0 | 2,0 | - | - | - | - | - | - |
| **Cutina**® **GMS** | - | - | - | - | - | 2,0 | 4,0 | - | - | 4,0 |
| Glyceryl Stearate | | | | | | | | | | |
| **Lanette**® **O** | - | - | 2,0 | - | 2,0 | 4,0 | 2,0 | 4,0 | 4,0 | 1,0 |
| Cetearyl Alcohol | | | | | | | | | | |
| **Antaron**® **V 216** | - | - | - | - | - | 3,0 | - | - | - | 2,0 |
| PVP / Hexadecene Copolymer | | | | | | | | | | |
| **Myritol**® **818** | 5,0 | - | 10,0 | - | 8,0 | 6,0 | 6,0 | - | 5,0 | 5,0 |
| Cocoglycerides | | | | | | | | | | |
| **Finsolv**® **TN** | - | 6,0 | - | 2,0 | - | - | 3,0 | - | - | 2,0 |
| C12/15 Alkyl Benzoate | | | | | | | | | | |
| **Cetiol**® **J 600** | 7,0 | 4,0 | 3,0 | 5,0 | 4,0 | 3,0 | 3,0 | - | 5,0 | 4,0 |
| Olcyl Erucate | | | | | | | | | | |
| **Cetiol**® **OE** | 3,0 | - | 6,0 | 8,0 | 6,0 | 5,0 | 4,0 | 3,0 | 4,0 | 6,0 |
| Dicaprylyl Ether | | | | | | | | | | |
| **Mineral Oil** | - | 4,0 | - | 4,0 | - | 2,0 | - | 1,0 | - | - |
| **Cetiol**® **PGL** | - | 7,0 | 3,0 | 7,0 | 4,0 | - | - | - | 1,0 | - |
| Hexadecanol (and) Hexyldecyl Laurate | | | | | | | | | | |
| Bisabolol | 1,2 | 1,2 | 1,2 | 1,2 | 1,2 | l,2 | 1,2 | 1,2 | l,2 | 1,2 |
| **Retinol-Mikrokapseln nach Beispiel 1** | 1,0 | 1,0 | 1,0 | 1,0 | 1,0 | 1,0 | 1,0 | 1,0 | 1,0 | 1,0 |
| **Hydagen**® **CMF** | 1,0 | 1,0 | 1,0 | 1,0 | 1,0 | 1,0 | 1,0 | 1,0 | 1,0 | 1,0 |
| Chitosan | | | | | | | | | | |
| **Copherol**® **F 1300** | 0,5 | 1,0 | 1,0 | 2,0 | 1,0 | 1,0 | 1,0 | 2,0 | 0,5 | 2,0 |
| Tocopherol / Tocopheyl Acetate | | | | | | | | | | |
| Neo **Heliopan**® **Hydro** | 3,0 | - | - | 3,0 | - | - | 2,0 | - | 2,0 | - |
| Sodium Phenylbenzimidazole Sulfonate | | | | | | | | | | |
| Neo **Heliopan**® **303** | - | 5,0 | - | - | - | 4,0 | 5,0 | - | - | 10,0 |
| Octocrylene | | | | | | | | | | |
| Neo **Heliopan**® **BB** | 1,5 | - | - | 2,0 | 1,5 | - | - | - | 2,0 | - |
| Benzophenone-3 | | | | | | | | | | |
| **Neo Heliopan**® **E 1000** | 5,0 | - | 4,0 | - | 2,0 | 2,0 | 4,0 | 10,0 | - | - |
| Isoamyl p-Methoxycinnamate | | | | | | | | | | |
| Neo **Heliopan**® **AV** | 4,0 | - | 4,0 | 3,0 | 2,0 | 3,0 | 4,0 | - | 10,0 | 2,0 |
| Octyl Methoxycinnamate | | | | | | | | | | |
| **Uvinul**® **T 150** | 2,0 | 4,0 | 3,0 | 1,0 | 1,0 | 1,0 | 4,0 | 3,0 | 3,0 | 3,0 |
| Octyl Triazone | | | | | | | | | | |
| **Zinc Oxide** | - | 6,0 | 6,0 | - | 4,0 | - | - | - | - | 5,0 |
| **Titanium Dioxide** | - | - | - | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5,0 |

| | | | | | | | | | | |
|---|---|---|---|---|---|---|---|---|---|---|
| (31) W/O-Sonnenschutzcreme, (32-34) W/O-Sonnenschutzlotion, (35, 38, 40) O/w-Sonnenschutzlotion (36, 37,39) O/W-Sonnenschutzcreme | | | | | | | | | | |

## Patentansprüche

1. Mikrokapseln mit mittleren Durchmessern im Bereich von 0,0001 bis 5 mm, bestehend aus einer Hüllmembran und einer die Wirkstoffe enthaltenden Matrix, **dadurch** erhältlich, dass man
(a1) fettlösliche Wirkstoffe mit Schmelzpunkten unterhalb von 20 °C und Wachse mit Schmelzpunkten oberhalb von 20 °C zu einer Ölphase verarbeitet,
(a2) die so erhaltene Ölphase mit einer wässrigen Lösung eines Anionpolymers bei einer Temperatur oberhalb des Schmelzpunktes der Wachse vermischt, und
(a3) die so erhaltene O/W-Emulsion auf eine Temperatur unterhalb des Schmelzpunktes der Wachse abkühlt und mit der wässrigen Lösung eines Kationpolymers in Kontakt bringt,
oder
(b1) fettlösliche Wirkstoffe mit Schmelzpunkten unterhalb von 20 °C und Wachse mit Schmelzpunkten oberhalb von 20 °C zu einer Ölphase verarbeitet,
(b2) die so erhaltene Ölphase mit einer wässrigen Lösung eines Kationpolymers bei einer Temperatur oberhalb des Schmelzpunktes der Wachse vermischt, und
(b3) die so erhaltene O/W-Emulsion auf eine Temperatur unterhalb des Schmelzpunktes der Wachse abkühlt und mit der wässrigen Lösung eines Anionpolymers in Kontakt bringt.

2. Verfahren zur Herstellung von Mikrokapseln mit mittleren Durchmessern im Bereich von 0,0001 bis 5 mm, bestehend aus einer Hüllmembran und einer die Wirkstoffe enthaltenden Matrix, bei dem man
(a1) fettlösliche Wirkstoffe mit Schmelzpunkten unterhalb von 20 °C und Wachse mit Schmelzpunkten oberhalb von 20 °C zu einer Ölphase verarbeitet,
(a2) die so erhaltene Ölphase mit einer wässrigen Lösung eines Anionpolymers bei einer Temperatur oberhalb des Schmelzpunktes der Wachse vermischt, und
(a3) die so erhaltene O/W-Emulsion auf eine Temperatur unterhalb des Schmelzpunktes der Wachse abkühlt und mit der wässrigen Lösung eines Kationpolymers in Kontakt bringt,
oder
(b1) fettlösliche Wirkstoffe mit Schmelzpunkten unterhalb von 20 °C und Wachse mit Schmelzpunkten oberhalb von 20 °C zu einer Ölphase verarbeitet,
(b2) die so erhaltene Ölphase mit einer wässrigen Lösung eines Kationpolymers bei einer Temperatur oberhalb des Schmelzpunktes der Wachse vermischt, und
(b3) die so erhaltene O/W-Emulsion auf eine Temperatur unterhalb des Schmelzpunktes der Wachse abkühlt und mit der wässrigen Lösung eines Anionpolymers in Kontakt bringt.

3. Verfahren nach Anspruch 2, **dadurch gekennzeichnet, dass** man fettlösliche Wirkstoffe einsetzt, die ausgewählt sind aus der Gruppe, die gebildet wird von Retinol, Retinolsäure, Bisabolol, Panthenol, Tocopherolen, Carotinoiden, Flavonoiden und deren Gemischen.

4. Verfahren nach den Ansprüchen 2 und/oder 3, **dadurch gekennzeichnet, dass** man Wachse einsetzt, die ausgewählt sind aus der Gruppe die gebildet wird von Fettalkoholen, Wachsestern, Sterolen und deren Gemischen.

5. Verfahren nach mindestens einem der Ansprüche 2 bis 4, **dadurch gekennzeichnet, dass** man Anionpolymere einsetzt, die ausgewählt sind aus der Gruppe, die gebildet wird von Alginsäure und deren Salzen, Poly(meth)acrylaten, Carboxymethylcellulosen, anionischen Tensiden und niedermolekularen anorganischen Salzen.

6. Verfahren nach mindestens einem der Ansprüche 2 bis 5, **dadurch gekennzeichnet, dass** man die Anionpolymere zusammen mit Gelbildnern und/oder Emulgatoren einsetzt.

7. Verfahren nach Anspruch 6, **dadurch gekennzeichnet, dass** man als Gelbildner Heteropolysaccharide und/oder Proteine einsetzt.

8. Verfahren nach Anspruch 6, **dadurch gekennzeichnet, dass** man als Emulgatoren nichtionische Tenside einsetzt.

9. Verfahren nach mindestens einem der Ansprüche 2 bis 8, **dadurch gekennzeichnet, dass** man kationische Polymere einsetzt, die ausgewählt sind aus der Gruppe, die gebildet wird von kationischen Cellulosederivaten, kationischen Stärken, Copolymeren von Diallylammoniumsalzen und Acrylamiden, quaternierten Vinylpyrrolidon/Vinylimidazol-Polymeren, Kondensationsprodukten von Polyglycolen und Aminen, quaternierten Kollagenpolypeptiden, quaternierten Weizenpolypeptiden, Polyethyleniminen, kationischen Siliconpolymeren, Copolymeren der Adipinsäure und Dimethylaminohydroxypropyldiethylentriamin, Copolymeren der Acrylsäure mit Dimethyldiallylammoniumchlorid, Polyaminopolyamiden sowie deren vernetzten wasserlöslichen Polymeren, kationischen Chitinderivaten, Kondensationsprodukten aus Dihalogenalkylen mit Bisdialkylaminen, kationischem Guar-Gum sowie quaternierten Ammoniumsalzpolymeren.

10. Verfahren nach mindestens einem der Ansprüche 2 bis 8, **dadurch gekennzeichnet, dass** man als kationische Polymere Chitosane einsetzt.

11. Verwendung von Mikrokapseln nach Anspruch 1 zur Herstellung von kosmetischen Zubereitungen.

12. Verwendung von Mikrokapseln nach Anspruch 1 zur Ausrüstung von Textilien.

## Claims

1. Microcapsules with average diameters in the range from 0.0001 to 5 mm, consisting of an encapsulation membrane and a matrix containing the active ingredients, which may be obtained
(a1) by transforming fat-soluble active ingredients having melting points less than 20°C and waxes having melting points above 20°C into an oil phase,
(a2) by mixing the thereby obtained oil phase with an aqueous solution of an anionic polymer at a temperature above the melting point of the waxes, and
(a3) by cooling the thereby obtained oil-in-water emulsion to a temperature below the melting point of the waxes and by bringing it into contact with the aqueous solution of a cationic polymer,
or
(b1) by transforming fat-soluble active ingredients having melting points below 20°C and waxes having melting points above 20°C into an oil phase,
(b2) by mixing the thereby obtained oil phase with an aqueous solution of a cationic polymer at a temperature above the melting point of the waxes, and
(b3) by cooling the thereby obtained oil-in-water emulsion to a temperature below the melting point of the waxes and by bringing it into contact with the aqueous solution of a anionic polymer.

2. A method for producing microcapsules with average diameters in the range from 0.0001 to 5 mm, consisting of an encapsulation membrane and a matrix containing the active ingredients, wherein
(a1) fat-soluble active ingredients having melting points below 20°C and waxes having melting points above 20°C are transformed into an oil phase,
(a2) the thereby obtained oil phase is mixed with an aqueous solution of an anionic polymer at a temperature above the melting point of the waxes, and
(a3) the thereby obtained oil-in-water emulsion is cooled to a temperature below the melting point of the waxes and is brought into contact with the aqueous solution of an cationic polymer,
or
(b1) fat-soluble active ingredients having melting points above 20°C are transformed into an oil phase,
(b2) the thereby obtained oil phase is mixed with an aqueous solution of a cationic polymer at a temperature above the melting point of the waxes, and
(b3) the thereby obtained oil-in-water emulsion is cooled to a temperature below the melting point of the waxes and is brought into contact with the aqueous solution of an anionic polymer.

3. The method according to claim 2, **characterized in that** fat-soluble active ingredients are applied, which are selected from the group formed by retinol, retinoic acid, bisabolol, panthenol, tocopherols, carotenoids, flavonoids and mixtures thereof.

4. The method according to claim 2 and/or 3, **characterized in that** waxes are applied which are selected from the group which is formed by fatty alcohols, wax esters, sterols and mixtures thereof.

5. The method according to at least one of claims 2 to 4, **characterized in that** anionic polymers are applied, which are selected from the group formed by alginic acid and its salts, poly(meth)acrylates, carboxymethyl celluloses, anionic surfactants, and low molecular weight inorganic salts.

6. The method according to at least one of claims 2 to 5, **characterized in that** anionic polymers are applied together with gelling agents and/or emulsifiers.

7. The method according to claim 6, **characterized in that** heteropolysaccharides and/or proteins are applied as gelling agents.

8. The method according to claim 6, **characterized in that** non-ionic surfactants are applied as emulsifiers.

9. The method according to at least one of claims 2 to 8, **characterized in that** cationic polymers are applied, which are selected from the group formed by cationic cellulose derivatives, cationic starches, copolymers of diallylammonium salts and acrylamides, quaternized vinylpyrrolidonevinylimidazole polymers, condensation products of polyglycols and amines, quaternized collagen polypeptides, quaternized wheat polypeptides, polyethylene imines, cationic silicone polymers, copolymers of adipic acid and of dimethylaminohydroxypropyldiethylene-triamine, copolymers of acrylic acid with dimethyldiallylammonium chloride, polyaminopolyamides as well as their cross-linked water-soluble polymers, cationic chitin derivatives, condensation products of dihaloalkanes with bisdialkylamines, cationic guar gum as well as quaternized ammonium salt polymers.

10. The method according to at least one of claims 2 to 8, **characterized in that** chitosans are applied as cationic polymers.

11. The use of microcapsules according to claim 1 for producing cosmetic preparations.

12. The use of microcapsules according to claim 1 for finishing textiles.

## Revendications

1. Microcapsules ayant des diamètres moyens dans la plage de 0,0001 à 5 mm, constituées d'une membrane d'enveloppe et d'une matrice contenant les substances actives, que l'on peut obtenir
(a1) en transformant des substances actives liposolubles ayant des points de fusion inférieurs à 20°C et des cires ayant des points de fusion supérieurs à 20°C en une phase d'huile,
(a2) en mélangeant la phase d'huile ainsi obtenue avec une solution aqueuse d'un polymère anionique à une température supérieure au point de fusion des cires, et
(a3) en refroidissant l'émulsion huile-dans-eau ainsi obtenue à une température inférieure au point de fusion des cires et en l'amenant en contact avec la solution aqueuse d'un polymère cationique,
ou
(b1) en transformant des substances actives liposolubles ayant des points de fusion inférieurs à 20°C et des cires ayant des points de fusion supérieurs à 20°C en une phase d'huile,
(b2) en mélangeant la phase d'huile ainsi obtenue avec une solution aqueuse d'un polymère cationique à une température supérieure au point de fusion des cires, et
(b3) en refroidissant l'émulsion huile-dans-eau ainsi obtenue à une température inférieure au point de fusion des cires et en l'amenant en contact avec la solution aqueuse d'un polymère anionique.

2. Procédé pour produire des microcapsules ayant des diamètres moyens dans la plage de 0,0001 à 5 mm, constituées d'une membrane d'enveloppe et d'une matrice contenant les substances actives, dans lequel
(a1) on transforme des substances actives liposolubles ayant des points de fusion inférieurs à 20°C et des cires ayant des points de fusion supérieurs à 20°C en une phase d'huile,
(a2) on mélange la phase d'huile ainsi obtenue avec une solution aqueuse d'un polymère anionique à une température supérieure au point de fusion des cires, et
(a3) on refroidit l'émulsion huile-dans-eau ainsi obtenue à une température inférieure au point de fusion des cires et on l'amène en contact avec la solution aqueuse d'un polymère cationique,
ou
(b1) on transforme des substances actives liposolubles ayant des points de fusion supérieurs à 20°C en une phase d'huile,
(b2) on mélange la phase d'huile ainsi obtenue avec une solution aqueuse d'un polymère cationique à une température supérieure au point de fusion des cires, et
(b3) on refroidit l'émulsion huile-dans-eau ainsi obtenue à une température inférieure au point de fusion des cires et on l'amène en contact avec la solution aqueuse d'un polymère anionique.

3. Procédé selon la revendication 2, **caractérisé en ce que** l'on met en oeuvre des substances actives liposolubles, qui sont choisies dans le groupe qui est formé par le rétinol, l'acide rétinoïque, le bisabolol, le panthénol, les tocophérols, les caroténoïdes, les flavonoïdes et leurs mélanges.

4. Procédé selon les revendications 2 et/ou 3, **caractérisé en ce que** l'on met en oeuvre des cires qui sont choisies dans le groupe qui est formé par les alcools gras, les esters de cire, les stérols et leurs mélanges.

5. Procédé selon au moins l'une des revendications 2 à 4, **caractérisé en ce que** l'on met en oeuvre des polymères anioniques qui sont choisis dans le groupe qui est formé par l'acide alginique et ses sels, les poly(meth)acrylates, les carboxyméthylcelluloses, les tensio-actifs anioniques et les sels inorganiques de faible masse moléculaire.

6. Procédé selon au moins l'une des revendications 2 à 5, **caractérisé en ce que** l'on met en oeuvre les polymères anioniques conjointement avec des agents de gélification et/ou des émulsifiants.

7. Procédé selon la revendication 6, **caractérisé en ce que** l'on met en oeuvre les hétéropolysaccharides et/ou les protéines en tant qu'agents de gélification.

8. Procédé selon la revendication 6, **caractérisé en ce que** l'on met en oeuvre les tensio-actifs non ioniques en tant qu'émulsifiants.

9. Procédé selon au moins l'une des revendications 2 à 8, **caractérisé en ce que** l'on met en oeuvre des polymères cationiques qui sont choisis dans le groupe qui est formé par les dérivés cationiques de cellulose, les amidons cationiques, les copolymères de sels de diallylammonium et d'acrylamides, les polymères vinylpyrrolidone/vinylimidazole quaternisés, les produits de condensation de polyglycols et d'amines, les polypeptides de collagène quaternisés, les polypeptides de blé quaternisés, les polyéthylèneimines, les polymères cationiques de silicones, les copolymères de l'acide adipique et de la diméthylaminohydroxypropyldiéthylène-triamine, les copolymères de l'acide acrylique avec le chlorure de diméthyldiallylammonium, les polyaminopolyamides ainsi que leurs polymères hydrosolubles réticulés, les dérivés cationiques de chitine, les produits de condensation de dihalogénoalkylène avec les bisdialkylamines, la gomme de guar cationique ainsi que les polymères de sels d'ammonium quaternisés.

10. Procédé selon au moins l'une quelconque des revendications 2 à 8, **caractérisé en ce que** l'on met en oeuvre les chitosans en tant que polymères cationiques.

11. Utilisation de microcapsules selon la revendication 1 pour produire des préparations cosmétiques.

12. Utilisation de microcapsules selon la revendication 1 pour l'apprêt de textiles.
